# EUROPEAN PATENT SPECIFICATION

(11) **EP 4 017 280 B1**
(45) Date of publication and mention of the grant of the patent: **16.09.2026**
(21) Application number: 20829163.3
(22) Date of filing: 16.12.2020
(51) Int. Cl.: A23K 50/40, A23K 20/163, A23K 50/42, A61P 3/04

(54) **PET FOOD COMPOSITIONS**
HAUSTIERFUTTERMITTEL
COMPOSITIONS POUR ANIMAUX DE COMPANIE

(30) Priority: 27.12.2019 US 201962954050 P
(43) Date of publication of application: 29.06.2022
(73) Proprietor: Hill's Pet Nutrition, Inc., Topeka, KS 66603 (US)
(72) Inventor: JACKSON, Matthew, Topeka, KS 66604 (US); JEWELL, Dennis, Lawrence, KS 66049 (US)
(74) Representative: Wichmann, Hendrik
(86) International application number: PCT/US2020/065277
(87) International publication number: WO 2021/133602

(56) References cited:
- CZ-U1- 33 069
- JP-A- 2004 008 098
- US-A1- 2009 148 560
- US-A1- 2011 177 175
- US-A1- 2011 269 828
- US-A1- 2016 000 737
- DONADELLI RENAN A ET AL: "The effects on nutrient utilization and stool quality of Beagle dogs fed diets with beet pulp, cellulose, and Miscanthus grass12", JOURNAL OF ANIMAL SCIENCE, vol. 97, no. 10, 3 October 2019 (2019-10-03), pages 4134 - 4139, XP093031184, ISSN: 0021-8812, Retrieved from the Internet <URL:https://www.ncbi.nlm.nih.gov/pmc/articles/PMC6776306/pdf/skz265.pdf> DOI: 10.1093/jas/skz265

## Description

### BACKGROUND

Obesity is a problematic condition in pets and associated with a host of health problems such as inflammation and metabolic endotoxemia. One cause of obesity is overeating due to a lack of satiety. Treating obesity caused in such a manner has proven difficult. Pets require a significant amount of time to achieve weight and fat loss and transition from an obese body to a leaner and healthier body. A treatment plan that shortens this time period would be highly beneficial to pets and owners, particularly if the treatment improved the general satiety of a pet and secondary conditions associated with obesity such as metabolic endotoxemia and inflammation.

Current research has begun to show that improving the pet-microbiome relationship aids in the improvements of pet obesity and subsequent obesity-related conditions.

Thus, there is a need in the art for a pet food that aids in the management of obesity by increasing fat loss while maintaining satiety and also improves obesity-related conditions including metabolic endotoxemia, and reduced inflammation.
A feed for suppressing an increase in blood cholesterol level for livestock and pets with beet pulp is as a main fiber source is described in JP 2004 8098 A. A dog diet with beet pulp and its effects on nutrient utilization and stool quality of Beagle dogs is described in Donadelli et al., Journal of Animal Science, 2019, 4134-4139. A pet food for the prevention and management of obesity which comprises a diacylglycerol and a carbohydrate such as modified starch and fibre, is disclosed in US 2009 148560 A1.

### BRIEF SUMMARY

The present invention provides the pet food composition and medical uses thereof defined in the appended claims. The pet food composition has a weight ratio of NDF to crude fiber of at least about 3:2, and comprises fiber complex comprising pecan fiber, beet pulp and/or cranberry pomace. Some embodiments of the present invention provide a pet food composition comprising about a 2:1 weight ratio NDF to crude fiber. In other embodiments, the pet food composition comprises about a 4:1 weight ratio of NDF to crude fiber. In certain embodiments, the dietary fiber is chosen from a group consisting of pecan fiber, and beets. In certain embodiments, the total dietary fiber is greater than 30%.

In further embodiments, the NDF is greater than 20% wt. of the composition and/or the crude fiber is about 11% to about 20% wt. of the composition. In some embodiments, the pet food composition comprises about 21% NDF and about 13% crude fiber. Still further embodiments of the present invention comprise about 6% to about 12% moisture.

In some embodiments, the present invention comprises an animal feed and fiber bundle. The fiber bundle may be at a concentration of about 10% weight to about 50% by weight, of the pet food composition. In some embodiments, the animal feed is a commercially prepared pet food. The composition of the present invention may comprise a weight ratio of about 1:1 to about 1:10 of the fiber bundle to animal feed.

The composition of present invention may be used in a method for preventing or treating obesity in animals by increasing fat loss and satiety as well as decreasing obesity-related conditions including but not limited to, metabolic endotoxemia and inflammation. This method may comprise administering any of the compositions described herein to a pet in need thereof. In such uses , the food intake of the pet may be restricted.

### BRIEF DESCRIPTION OF THE DRAWINGS

FIG. 1 depicts an exemplary pet food formulation according to one embodiment.
FIG. 2 depicts data demonstrating the time-control of Example 1.
FIG. 3 depicts data demonstrating the decrease of inflammatory arachidonates through both phases of Example 1.
FIG. 4 depicts data demonstrating the improvement in endotoxemic status provided by an exemplary composition of the present invention.
FIG. 5 depicts data demonstrating an increase in anti-inflammatory cytokine IL-10.
FIG. 6 depicts data demonstrating a decrease in pro-inflammatory cytokine MCP-1.
FIG. 7 depicts data demonstrating an increase in satiety hormone pancreatic peptide.
FIG. 8 depicts data demonstrating a decrease in hunger hormone ghrelin.
FIG. 9 depicts data demonstrating a decrease in amount of total weight.
FIG. 10 depicts data demonstrating a decrease in amount of total fat.

### DETAILED DESCRIPTION

The present invention relates to the composition of pet food and medical uses thereof defined in the claims. Specifically, the composition may be use in subsequent methods to aid in the management of obesity and obesity-related conditions through, e.g., decreasing gut bacterial inflammatory endotoxin in host circulation and/or decreasing host inflammation.

The following description of the preferred embodiment(s) is merely exemplary in nature and is in no way intended to limit the invention. The description of illustrative embodiments is intended to be read in connection with the accompanying drawings, which are to be considered part of the entire written description.

As used herein and in the appended claims, the singular forms "a", "an", and "the" include plural references unless the context dictates otherwise. The singular form of any class of the ingredients refers not only to one chemical species within that class, but also to a mixture of those chemical species; for example, the term "protein" in the singular form, may refer to a mixture of compounds each of which is also considered a protein. The terms "a" (or "an"), "one or more" and "at least one" may be used interchangeably herein. The terms "comprising", "including", and "having" may be used interchangeably. The term "include" should be interpreted as "include, but are not limited to". The term "including" should be interpreted as "including, but are not limited to".

As used throughout, ranges are used as shorthand for describing each and every value that is within the range. Any value within the range can be selected as the terminus of the range.

As used herein, the term "pet" could be used interchangeably with "companion animal" and refers to an animal of any species kept by a caregiver as a pet or any animal of a variety of species that have been widely domesticated as pets, including canines *(Canis familiaris*) and felines *(Felis domesticus).* Thus, a pet may include but is not limited to, working dogs, pet dogs, cats kept for rodent control (i.e. farm cats), pet cats, ferrets, birds, reptiles, rabbits, and fish.

The term "fiber", as used herein, could be used interchangeably with "dietary fiber" or "total dietary fiber" and may refer to soluble fiber, insoluble fiber, or a combination of both. Dietary fiber refers to components of a plant which are resistant to digestion by an animal's digestive enzymes.

As used herein, the phrase "soluble fiber" refers to dietary fiber that attracts water during digestion and slows the rate of nutrient absorption. Soluble fiber is resistant to digestion and absorption in the small intestine and undergo complete or partial fermentation in the large intestine, and is typically found in various plant sources, including oat bran, seeds, beans, and certain fruits and vegetables such as beet pulp, guar gum, chicory root, psyllium, pectin, blueberry, cranberry, squash, apples, oats, beans, citrus, barley and peas. The phrase encompasses any source of soluble fiber suitable for the compositions disclosed herein as would be evident to one of skill in the art.

Insoluble fiber may be supplied by any of a variety of sources, including cellulose, whole wheat products, wheat, oat, corn bran, flax seed, grapes, celery, green beans, cauliflower, potato skins, fruit skins, vegetable skins, peanut hulls, and soy fiber.

Crude fiber includes indigestible components contained in cell walls and cell contents of plants such as grains, e.g., hulls of grains such as wheat, oats, rice, corn, and beans.

Examples of fiber sources may comprise apples, apple pomace, barely, beans, beets, beet pulp, dried beet fiber (sugar removed), beta-glucans, blueberry, cauliflower, carrageenan, reduced starch, celery, cellulose, a-cellulose, carboxymethylcellulose, hemisellulose, chicory root, chitins, citrus, citrus pulp, citrus fiber, citrus pectin, cranberry, corn, corn bran, fiber extracts, fiber derivatives, flax seed, grapes, green beans, guar gum, xanthan gum alginates, gum arabic, gum talha, inulin, lignin, oats, oat fiber, wheat oats pectin, peanut hulls, pecan shells, potato, potato skins, psyllium, squash, whole wheat products, soy, soy fiber, fruit skins, vegetable skins, galactooligosaccharides, gentioligosaccharide xylooligosaccharides, fructooligosaccharides, lactulose, mannanoligosaccharides, polydextrose, pectic oligosaccharide, soy oligosaccharides, oligodextran, trehalose, raffinose, stachyose, oligo derivatives from starch, and/or non-starch polysaccharides.

The term "palatability", as used herein, encompasses all the various properties of food sensed by animals such as texture, taste and aroma. In certain embodiments, the composition has a palatability equal to that of a control composition.

The term "probiotic" may refer to any microorganism suitable for pet consumption and effective for improving the microbial balance in the pet gastrointestinal tract or for other benefits, such as disease or condition relief or prophylaxis, to the pet.

The term "kibble", as used herein, includes a particulate pellet like component of animal feeds, such as dog and cat feeds, typically having a moisture, or water, content of less than 12% by weight. Kibbles may range in texture from hard to soft and/or may range in internal structure from expanded to dense. Kibbles may be formed by an extrusion process. In nonlimiting examples, a kibble can be formed from a core and a coating to form a kibble that is coated, also called a coated kibble. It should be understood that when the term "kibble" is used, it can refer to an uncoated kibble or a coated kibble.

In some embodiments, the pet food composition is in the form of a kibble. In other embodiments, the pet food composition is in the form of multi-layer kibble and/or a multi-layer kibble comprising a coating. Further, the coating could comprise a palatant. In certain embodiments, the kibble is formed by extrusion. In other embodiments, the composition is in a form selected from: a loaf, a stew, a "meat and gravy" form, a gruel, shreds with a moisture content greater than 50%", and a product that could be pushed through a syringe. In another embodiment, the present invention comprises 6% wt. to about 12% wt. moisture.

In some embodiments, the kibble may comprise a binder. In certain embodiments the binder includes but is not limited to any of the following or combinations of the following: monosaccharides such as glucose, fructose, mannose, arabinose; di- and trisaccharides such as sucrose, lactose, maltose, trehalose, lactulose; corn and rice syrup solids; dextrins such as corn, wheat, rice and tapioca dextrins; maltodextrins; starches such as rice, wheat, corn, potato, tapioca starches, or these starches modified by chemical modification; alginates, chitosans; gums such as carrageen, and gum arabic; polyols such as glycerol, sorbitol, mannitol, xylitol, erythritol; esters of polyols such as sucrose esters, polyglycol esters, glycerol esters, polyglycerol esters, sorbitan esters; sorbitol; molasses; honey; gelatins; peptides; proteins and modified proteins such as whey liquid, whey powder, whey concentrate, whey isolate, whey protein isolate, high lactose whey by-product, meat broth solids such as chicken broth, chicken broth solids, soy protein, and egg white.

In certain embodiments, the binder includes but is not limited to a lipid and/or lipid derivative. Lipids can be used in combination with water and/or other binder components. Lipids can include plant fats such as soybean oil, corn oil, rapeseed oil, olive oil, safflower oil, palm oil, coconut oil, palm kernel oil, and partially and fully hydrogenated derivatives thereof; animal fats and partially and fully hydrogenated derivatives thereof; and waxes.

In certain embodiments, the present invention may comprise additional ingredients including but not limited to, additives, minerals, vitamins, sources of carbohydrates, fat, protein, additional fiber, amino acids, carotenoids, antioxidants, fatty acids, glucose mimetics, probiotics, prebiotics, and others.

The pet food composition may contain additives known in the art. Such additives should be present in amounts that do not impair the purpose and effect provided by the invention. Examples of additives include substances with a stabilizing effect, organoleptic substances, processing aids, and substances that provide nutritional benefits.

Stabilizing substances may increase the shelf life of the composition. Suitable examples can include preservatives, antioxidants, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches.

Additives for coloring, palatability, and nutritional purposes can include colorants, salts (including but not limited to sodium chloride, potassium citrate, potassium chloride, and other edible salts), vitamins, minerals, and flavoring. The amount of such additives in a composition typically is up to about 5% by weight (on a dry matter basis of the composition). Other additives can include antioxidants, omega-3 fatty acids, omega-6 fatty acids, glucosamine, chondroitin sulfate, vegetable extracts, herbal extracts, etc.

In certain embodiments, the pet food composition comprises vitamins and minerals in amounts required to avoid deficiency and maintain health. These amounts are readily available in the art. The Association of American Feed Control Officials (AAFCO) provides recommended amounts of such ingredients for dogs and cats *(see* Association of American Feed Control Officials. Official Publication, pp. 126-140 (2003)).

Vitamins could as an example include vitamin A, vitamin B1 (thiamine or related sources such as thiamine mononitrate), vitamin B2 (riboflavin), vitamin B3 (niacin), vitamin B5 (pantothenic acid or related sources such as calcium pantothenate), vitamin B6 (pyridoxine or related sources such as pyridoxine hydrochloride), vitamin B8 (folic acid), vitamin B12, vitamin C (ascorbic acid), vitamin D (such as a vitamin D3 supplements), vitamin E, vitamin H (biotin), vitamin K, acetate, choline and choline related sources such as choline chloride, and inositol.

Minerals and trace elements could as an example include calcium, phosphorus, sodium, potassium, magnesium, copper, zinc, choline, and iron salts. Mineral sources can include, for example, sodium selenite, monosodium phosphate, calcium carbonate, potassium chloride, ferrous sulfate, zinc oxide, manganese sulfate, copper sulfate, manganous oxide, potassium iodide, and/or cobalt carbonate.

The term "carbohydrate" as used herein includes polysaccharides (*e.g*., starches and dextrins) and sugars (*e.g*., sucrose, lactose, maltose, glucose, and fructose) that are metabolized for energy when hydrolyzed. Examples of carbohydrates suitable for inclusion in the compositions disclosed herein include but are not limited to, corn, grain sorghum, wheat, barley, and rice.

In certain embodiments, the carbohydrate component comprises a mixture of one or more carbohydrate sources. Examples of carbohydrate or carbohydrate ingredients may comprise cereals, grains, corn, wheat, rice, oats, corn grits, sorghum, grain sorghum/milo, wheat bran, oat bran, amaranth, Durum, and/or semolina.

One skilled in the art could manipulate the texture of the final product by properly balancing carbohydrate sources. For example, short chain polysaccharides tend to be sticky and gluey, and longer chain polysaccharides are less sticky and gluey than the shorter chain; the desired texture of this hybrid food is achieved by longer chain polysaccharide and modified starches such as native or modified starches, cellulose and the like.

The carbohydrate mixture may additionally comprise optional components such as added salt, spices, seasonings, vitamins, minerals, flavorants, colorants, and the like. The amount of the optional additives is at least partially dependent on the nutritional requirements for different life stages of animals.

The pet food composition of the present invention comprises about 10% wt. to about 14% wt. of fat. Sources of fats or fat ingredients, may comprise poultry fat, chicken fat, turkey fat, pork fat, lard, tallow, beef fat, vegetable oils, corn oil, soy oil, cottonseed oil, palm oil, palm kernel oil, linseed oil, canola oil, rapeseed oil, fish oil, menhaden oil, anchovy oil, and/or olestra.

The pet food composition of the present invention comprises about 15% wt. to about 20% wt. of protein. The term "protein" means a polypeptide, or a peptide, or a polymer of amino acids. The term encompasses naturally occurring and non-naturally occurring (synthetic) polymers and polymers in which artificial chemical mimetics are substituted for one or more amino acids. The term also encompasses fragments, variants, and homologs that have the same or substantially the same properties and perform the same or substantially the same function as the original sequence. The term encompasses polymers of any length, including polymers containing from about 2 to 1000, from 4 to 800, from 6 to 600, and from 8 to 400 amino acids. The term includes amino acid polymers that are synthesized and that are isolated and purified from natural sources. Under some embodiments, the terms "polypeptide", "peptide" or "protein" are used interchangeably.

Protein may be supplied by any of a variety of sources known by those of ordinary skill in the art including plant sources, animal sources, or both. For example, animal sources may include meat, meat-by products, seafood, dairy, eggs, etc. Meats, for example, may include animal flesh such as poultry fish, and mammals including cattle, pigs, sheep, goats, and the like. Meat by-products may include, for example, lungs, kidneys, brain, livers, stomachs and intestines. Plant protein includes, for example, soybean, corn, rice, cottonseed, and peanuts.

Examples of protein or protein ingredients may comprise chicken meals, chicken, chicken by-product meals, lamb, lamb meals, turkey, turkey meals, beef, beef by-products, viscera, fish meal, enterals, kangaroo, white fish, venison, soybean meal, soy protein isolate, soy protein concentrate, corn gluten meal, corn protein concentrate, distillers dried grains, and/or distillers dried grain solubles and single-cell proteins, for example yeast, algae, and/or bacteria cultures.

The protein can be intact, completely hydrolyzed, or partially hydrolyzed. The protein content of foods may be determined by any number of methods known by those of skill in the art, for example, as published by the Association of Official Analytical Chemists in Official Methods of Analysis ("OMA"), method 988.05. The amount of protein in a composition disclosed herein may be determined based on the amount of nitrogen in the composition according to methods familiar to one of skill in the art.

Examples of amino acids may comprise 1-Tryptophan, Taurine, Histidine, Carnosine, Alanine, Cysteine, Arginine, Methionine, Tryptophan, Lysine, Asparagine, Aspartic acid, Phenylalanine, Valine, Threonine, Isoleucine, Histidine, Leucine, Glycine, Glutamine, Taurine, Tyrosine, Homocysteine, Ornithine, Citruline, Glutamic acid, Proline, Serine and/or betaine. Sources of carotenoids may comprise lutein, astaxanthin, zeaxanthin, bixin, lycopene, and/or beta-carotene. Sources of antioxidant ingredients may comprise tocopherols (vitamin E), vitamin C, vitamin A, lipoic acid, plant-derived materials, carotenoids (described above), selenium, and/or CoQ10 (Co-enzyme Q10).

Examples of fatty acid ingredients may comprise arachidonic acid, alphalinoleic acid, gamma linolenic acid, linoleic acid, eicosapentanoic acid (EPA), docosahexanoic acid (DHA), and/or fish oils as a source of EPA and/or DHA. Sources of glucose mimetics may comprise glucose anti-metabolites including 2-deoxy Dglucose, 5-thio-D-glucose, 3-O-methylglucose, anhydrosugars including 1,5-anhydro-D-glucitol, 2,5-anhydro-D-glucitol, and 2,5-anhydro-D-mannitol, mannoheptulose, and/or avocado extract comprising mannoheptulose.

Still other ingredients may include beef broth, brewers dried yeast, egg, egg product, flax meal, DL methionine, amino acids, leucine, lysine, arginine, cysteine, cystine, aspartic acid, polyphosphates, sodium pyrophosphate, sodium tripolyphosphate; zinc chloride, copper gluconate, stannous chloride, stannous fluoride, sodium fluoride, triclosan, glucosamine hydrochloride, chondroitin sulfate, green lipped mussel, blue lipped mussel, methyl sulfonyl methane (MSM), boron, boric acid, phytoestrogens, phytoandrogens, genistein, diadzein, Lcarnitine, chromium picolinate, chromium tripicolinate, chromium nicotinate, acid/base modifiers, potassium citrate, potassium chloride, calcium carbonate, calcium chloride, sodium bisulfate; eucalyptus, lavender, peppermint, plasticizers, colorants, flavorants, sweeteners, buffering agents, slip aids, carriers, pH adjusting agents, natural ingredients, stabilizers, biological additives such as enzymes (including proteases and lipases), chemical additives, coolants, chelants, denaturants, drug astringents, emulsifiers, external analgesics, fragrance compounds, humectants, opacifying agents (such as zinc oxide and titanium dioxide), antifoaming agents (such as silicone), preservatives (such as butylated hydroxytoluene (BHT) and butylated hydroxyanisole (BHA), propyl gallate, benzalkonium chloride, EDTA, benzyl alcohol, potassium sorbate, parabens and mixtures thereof), reducing agents, solvents, hydrotropes, solubilizing agents, suspending agents (non-surfactant), solvents, viscosity increasing agents (aqueous and non-aqueous), sequestrants, and/or keratolytics.

The probiotic component may comprise any suitable bacteria, yeast, microorganisms, and/or mixtures of any thereof. Various probiotic microorganisms are known in the art. In certain embodiments, the probiotic component may comprise bacteria of the order *Lactobacillales;* bacteria of the genus *Bacillus, Bacteroides,* and/or *Bifidobacterium;* yeast of the order *Saccharomycetales* including the genus *Saccharomyces* and *Candida;* and/or mixtures of any thereof. The probiotic may or may not form a spore.

Non-limiting examples of bacteria of the order *Lactobacillales* suitable for use herein include the genus *Streptococci* such as *Streptococcus lactis, Streptococcus cremoris, Streptococcus diacetylactis,* and/or *Streptococcus thermophilus;* the genus *Enterococcus* such as *Enterococcus faecium,* the family *Lactobacillillaceae* including the genus *Pediococcus* (i.e. *Pediococcus cerevisiae),* the genus *Leuconostroc,* and the genus *Lactobacilli* such as *Lactobacillus bulgaricus, Lactobacillus acidophilus, Lactobacillus helveticus, Lactobacillus bifidus, Lactobacillus casei, Lactobacillus lactis, Lactobacillus plantarum, Lactobacillus rhamnosus, Lactobacillus delbrukii, Lactobacillus thermophilus, Lactobacillus fermentii, Lactobacillus salvarius, Lactobacillus reuteri,* and/or mixtures of any thereof. Nonlimiting examples of bacteria of the genus *Bifidobacteria* include *Bifidobacterium longum, Bifidobacterium infantis, Bifidobacterium bifidum, Bifidobacterium animalis,* and/or *Bifidobacterium pseudolongum;* and/or mixtures of any thereof.

In certain embodiments, the pet food composition may include polyphenols. In some embodiments, the polyphenol source comprises a phenolic compound selected from ellagic acid; gallic acid; protocatechuic acid; p-hydroxybenzoic acid; catechin; and a combination of two or more thereof. In some embodiments, the polyphenol source comprises pecan shells, or any other component of the pecan nut. In some embodiments, the pecan shell may also be a source of lignin-based fiber. Examples of further sources of polyphenols may comprise tea extract, rosemary extract, rosemarinic acid, coffee extract, pecan shells, caffeic acid, turmeric extract, blueberry extract, grape extract, grapeseed extract, and/or soy extract

The pet food composition may undergo feed analysis using any of the variety of methods known to one skilled in the art. Feed analysis may be done to measure any of the nutritional content listed herein including moisture, protein, fiber, carbohydrate, energy, vitamin, mineral, energy, fat, and ash content.

Protein content may be measured and reported in any of the variety of methods known to one skilled in the art. Protein may be reported as crude protein (CP) to measure both true protein content and non-protein nitrogen. Crude protein content may be further differentiated between degradable intake protein (DIP), undegradable intake protein (UIP) and metabolizeable protein (MP). In certain embodiments, protein content may be differentiated to include heat damaged protein or insoluble crude protein (ICP), adjusted crude protein (ACP), and digestible protein (DP).

Fiber content may be measured and reported in any of the variety of methods known to one skilled in the art. Fiber content may be reported as crude fiber (CF), neutral detergent fiber (NDF), acid detergent fiber (ADF) and/or acid detergent lignin (ADL). Crude fiber is generally known to measure the indigestible portion of plant material found in pet food compositions. ADF measures cellulose and lignin, components of plant cell walls. NDF measures the total material found in plant cell walls and includes hemicellulose in addition to the fiber content measured as ADF. ADL measures only the lignin portion of a plant cell wall.

Energy content may be measured and reported in any of the variety of methods known to one skilled in the art. Energy content may be reported as total digestible nutrient (TDN), net energy (NE), metabolizable energy (ME), relative feed value (RFV), and relative forage quality (RFQ).

The pet food composition of the present invention has a weight ratio of NDF to crude fiber of at least about 3:2, and comprises fiber complex comprising pecan fiber, beet pulp and/or cranberry pomace. In embodiments, the composition of the present invention may comprise of a 2:1 ratio of NDF to crude fiber. Dietary fiber may be chosen from a group consisting of pecan fiber, and beets. The content of NDF fiber is of greater than about 15% and the content of crude fiber is of greater than about 10 wt%. In certain embodiments, the total dietary fiber is greater than 30%. In further embodiments, the NDF is greater than 20% wt. of the composition and/or the crude fiber is about 11% to about 20% wt. of the composition. In some embodiments, the pet food composition comprises about 21% NDF and about 13% crude fiber.

In certain embodiments of the present invention, the pet food composition may achieve any of the desired effects listed herein through the addition of a "fiber bundle" or "bundle" of ingredients. In certain embodiments, the fiber bundle comprises about 0.1% wt. to about 10% wt. of each ingredient. Ingredients for the bundle may include buckwheat groats, oats, pecan fiber, oat bran, fish oil, beet pulp, cranberry pomace, ginger root, yeast cell walls, pomegranate extract, green tea, and curcumin.

In one embodiment of the present invention, the pet food is comprised of at least 25% wt. of the fiber bundle. In another embodiment, the pet food composition comprises 100% of the fiber bundle. In yet another embodiment, the pet food composition may comprise between 10% and 50% of the fiber bundle.

The pet food composition may be used as a supplement to other pet foods known by one skilled in the art. The pet food composition may be added to another pet food from a 1:1 ratio to a 1:10 ratio, preferably at a ratio of 1:4.

The pet food composition of the present invention may be used in a method for preventing or treating obesity in animals or obesity-related conditions including but not limited to, metabolic endotoxemia and inflammation. This method may comprise administering any of the compositions described herein to a pet in need thereof.

In some embodiments, food intake is calculated based upon intake of calories normalized to metabolic bodyweight.

Embodiments of the present invention will now be further described by way of the following, non-limiting, example.

### EXAMPLE

### Example 1

Diets were formulated according to AAFCO (American Association of Feed Control Officials) and NRC (National Research Council) nutrition recommendations. The finished kibble was produced by extrusion, dried, and coated with palatants. All diets were canine maintenance formulations.

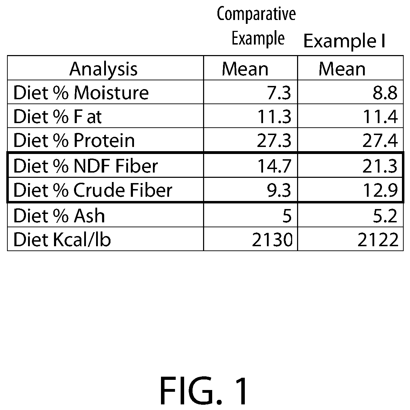

Referring to FIG. 1, the control diet (hereinafter "Comparative Example") contained only the components of the nutrition recommendations. The inventive diet (hereinafter "Example I") contained an increased level of NDF and an increased level of crude fiber as compared to the Comparative Example.

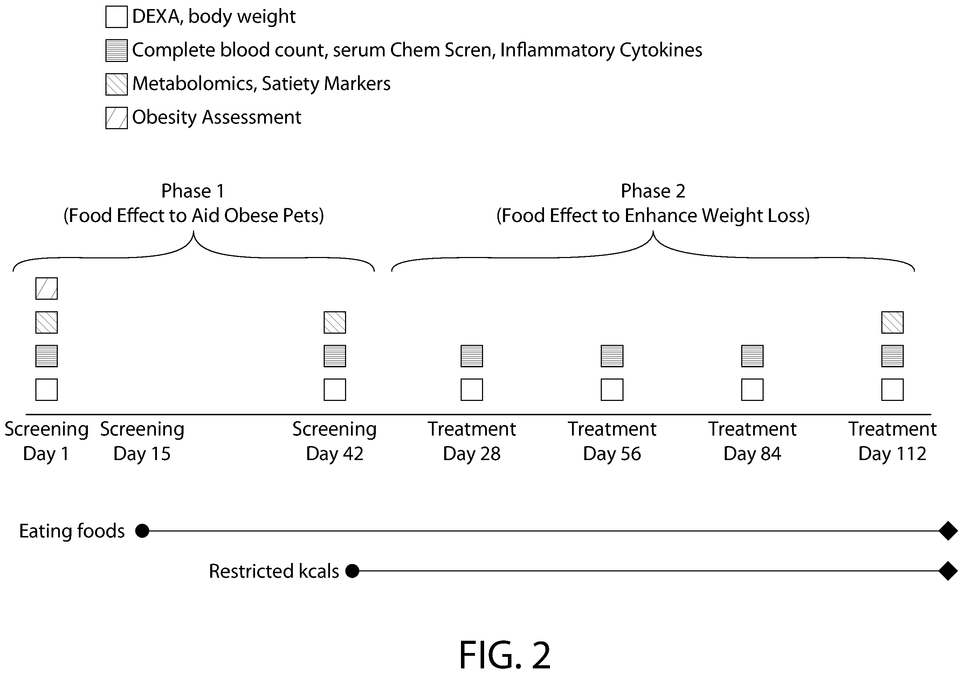

An IACUC approved clinical dietary intervention protocol was implemented which enrolled healthy canine subjects randomized to two groups based on age, weight, obesity status, and sex. The study was a caretaker- blinded, longitudinal design in two phases (see FIG. 2) conducted over about 150 days. Phase 1 consisted of baseline readings and the pets beginning a diet of the control or invention. Phase 2 consisted of the pets continuing the two diets and further, having their calories restricted.

Body composition and subsequent obesity status of each dog in the trial was assessed using duel-energy X-ray absorptiometry (DEXA). Blood draws for satiety hormones (ghrelin, pancreatic peptide), lipopolysaccharide (LPS), cytokines (IL-10 and MCP-1) and metabolomic assessment (proinflammaotry arachidonic acid metabolites) were performed immediately prior to DEXA analysis. Serum was lyophilized and extracted with methanol:water to liberate metabolites from serum matrix.

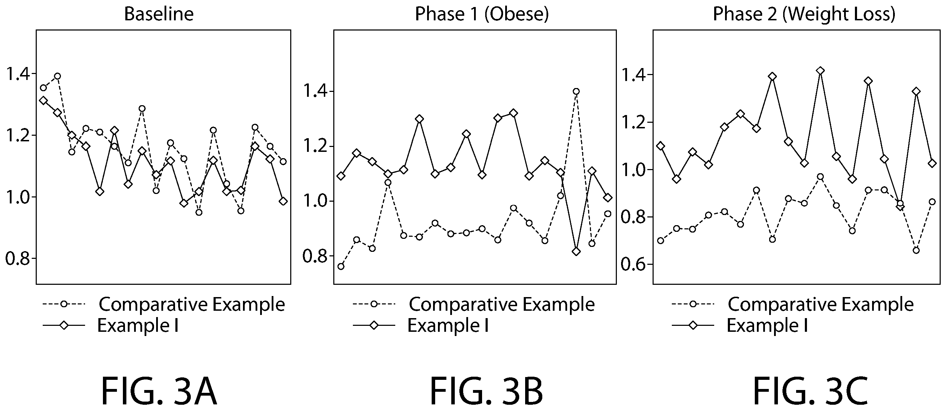

Metabolomics were performed by liquid chromatography-mass spectrometry (LS-MC) with relative fold quantitation. Circulating satiety hormones, LPS and cytokines were assessed by enzyme linked immunosorbent assay (ELISA) and expressed in expressed in picograms per milliliter (pg/ml). The total amount of weight lost and fat lost were calculated through DEXA analysis and expressed in kilograms (kg) and grams (g) respectively. Results are presented in FIGS. 3-9 and depict the relative levels of a given metabolite that was circulating in the dogs after being fed either a control or inventive pet food.

As shown in FIG. 3, an exemplary composition of the present invention decreased small molecule precursors of the prostaglandin pathway. This pathway composed of bound and circulating forms of arachidonic acid contains the precursors of inflammatory prostaglandins produced by cyclooxygenase enzymes. At baseline before feeding there was no significant difference in the arachidonate pathway between the Comparative Example and Example I dogs (see, FIG 3A). However, once the dogs began consuming the foods, a decrease in the levels of circulating pro-inflammatory arachidonic pathway metabolites was observed in the dogs fed an exemplary composition of the present invention, even before Phase 2 of the study (caloric restriction) was initiated (see, FIG. 3B). These benefits were also readily apparent after Phase 2 was completed (see, FIG. 3C). These results demonstrate that compositions of the present invention promote healthy weight management.

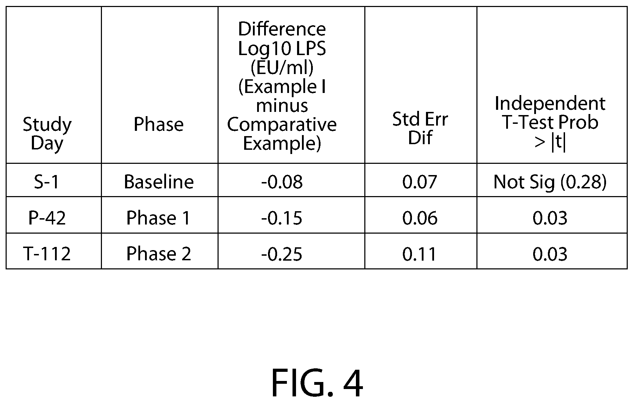

As the data described in FIG. 4 demonstrates, in Phase 1, before calorie restriction, an exemplary composition of the present invention decreased circulating bacterial lipopolysaccharide (LPS). Remarkably, this benefit was still present to a greater extent when compared to a comparative composition, after weight loss (Phase 2).

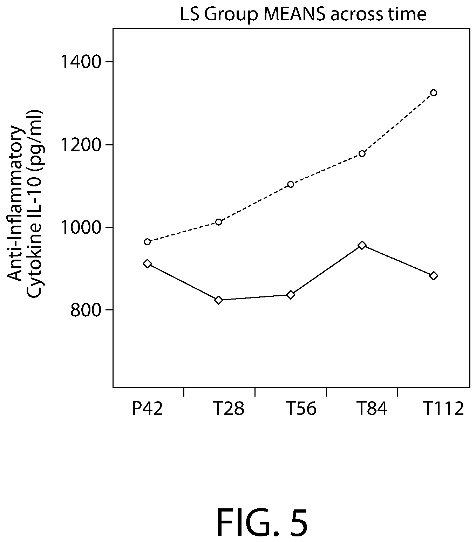

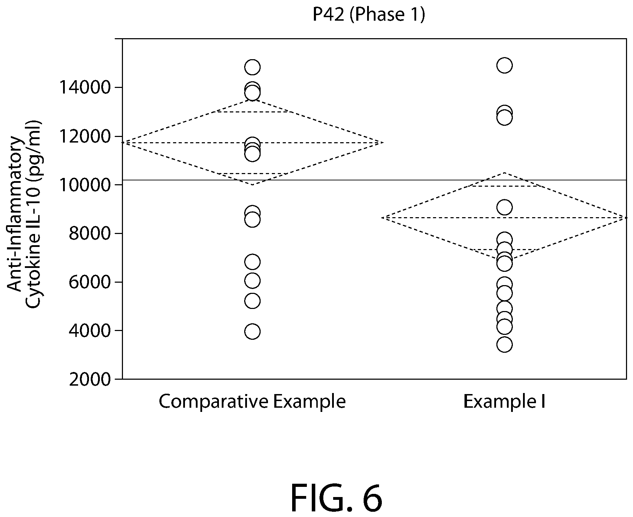

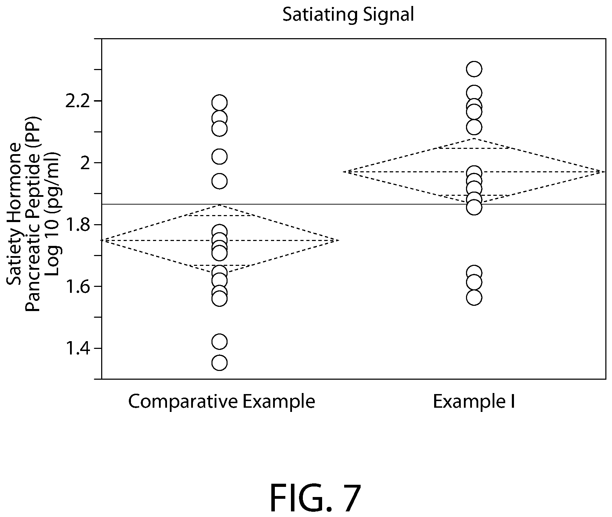

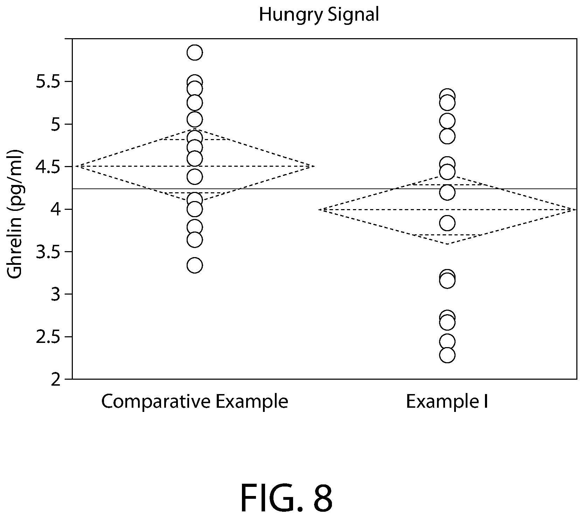

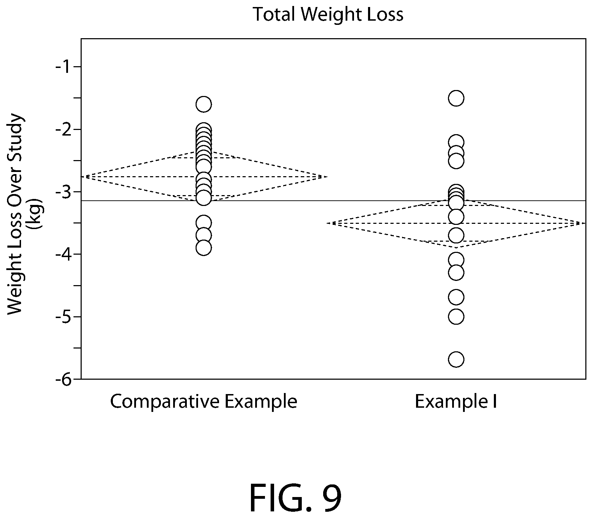

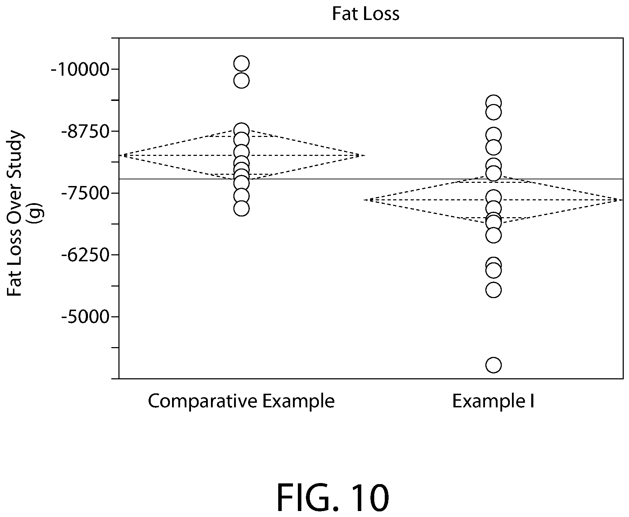

In addition to the dogs fed the invention losing a greater amount of total weight (see FIG. 9) and total fat (see FIG. 10) than the dogs fed the control, they also had a surprisingly greater reduction in obesity-related conditions. As depicted in FIG. 2, this benefit occurred throughout the entire trial during both phases. The invention significantly increased the amount of anti-inflammatory cytokine IL-10 (see, FIG. 5) and significantly reduced the amount of pro-inflammatory cytokine MCP-1 (see FIG. 6). In additional, the dogs fed the invention had greater satiety (see FIG. 7) and lower circulating hunger hormones (see FIG. 8).

## Claims

1. A pet food composition comprising:
a fiber complex comprising neutral detergent fiber (NDF) in an amount of greater than about 15 wt% and crude fiber in an amount of greater than about 10 wt%;
protein in an amount of about 15 wt% to about 20 wt%; and
fat in an amount of about 10 wt% to about 14 wt%;
wherein the weight ratio of NDF to crude fiber is at least about 3:2;
wherein the fiber complex comprises pecan fiber, beet pulp and/or cranberry pomace; and
wherein all weights are based on the total weight of the pet food composition.

2. The pet food composition according to claim 1, wherein the NDF comprises from about 55% to about 65% of the fiber complex.

3. The pet food composition according to claim 1 or claim 2, wherein the crude fiber comprises about from 35% to about 45% of the fiber complex.

4. The pet food composition according to any foregoing claim, wherein the fiber complex comprises greater than about 25%, optionally, greater than about 30%, based on the total weight of the pet food composition.

5. The pet food composition according to any foregoing claim, further comprising an anti-inflammatory agent; optionally, wherein the anti-inflammatory agent is selected from: curcumin; tetrahydrocurcumin; clove; ginger; rosemary; paprika; a berry (e.g. blueberry); and a combination of two or more thereof.

6. A pet food composition according to any one of claims 1-5 for use in a method for treating, preventing or ameliorating a symptom associated with obesity in a companion animal, optionally, wherein the symptom associated with obesity is selected from: joint pain; inflammation; and lethargy.

7. The pet food composition for use according to claim 6, wherein the symptom associated with obesity is treated, prevented or ameliorated by increasing pancreatic peptide in the companion animal.

8. A pet food composition according to any one of claims 1-5 for use in a method for treating, preventing, or reducing the risk of developing metabolic endotoxemia in a companion animal in need thereof.

9. The pet food composition for use according to any one of claims 6-8, wherein the companion animal is selected from a canine and a feline, preferably, a canine.

## Patentansprüche

1. Heimtierfutter-Zusammensetzung, umfassend:
einen Faser-Komplex, umfassend Neutral-Detergenzien-Faser (NDF) in einer Menge von mehr als etwa 15 Gew.-% und Rohfaser in einer Menge von mehr als etwa 10 Gew.-%;
Protein in einer Menge von etwa 15 Gew.-% bis etwa 20 Gew.-%; und
Fett in einer Menge von etwa 10 Gew.-% bis etwa 14 Gew.-%;
wobei das Gewichtsverhältnis von NDF zu Rohfaser mindestens etwa 3:2 beträgt;
wobei der Faser-Komplex Pekanfaser, Rübenschnitzel und/oder Cranberry-Trester umfasst; und
wobei alle Gewichte auf das Gesamtgewicht der Heimtierfutter-Zusammensetzung bezogen sind.

2. Heimtierfutter-Zusammensetzung nach Anspruch 1, wobei die NDF etwa 55% bis etwa 65% des Faser-Komplexes ausmacht.

3. Heimtierfutter-Zusammensetzung nach Anspruch 1 oder 2, wobei die Rohfaser etwa 35% bis etwa 45% des Faser-Komplexes ausmacht.

4. Heimtierfutter-Zusammensetzung nach einem der vorhergehenden Ansprüche, wobei der Faser-Komplex mehr als etwa 25%, optional mehr als etwa 30%, bezogen auf das Gesamtgewicht der Heimtierfutter-Zusammensetzung, ausmacht.

5. Heimtierfutter-Zusammensetzung nach einem der vorhergehenden Ansprüche, ferner umfassend ein entzündungshemmendes Mittel; optional, wobei das entzündungshemmende Mittel ausgewählt ist aus: Curcumin; Tetrahydrocurcumin; Gewürznelke; Ingwer; Rosmarin; Paprika; einer Beere (z. B. Heidelbeere); und einer Kombination von zwei oder mehr davon.

6. Heimtierfutter-Zusammensetzung nach einem der Ansprüche 1-5 zur Verwendung in einem Verfahren zum Behandeln, Verhindern oder Lindern eines mit Adipositas assoziierten Symptoms bei einem Begleittier, optional, wobei das mit Adipositas assoziierte Symptom ausgewählt ist aus: Gelenkschmerz; Entzündung; und Lethargie.

7. Heimtierfutter-Zusammensetzung zur Verwendung nach Anspruch 6, wobei das mit Adipositas assoziierte Symptom durch Erhöhen des Pankreas-Peptids in dem Begleittier behandelt, verhindert oder gelindert wird.

8. Heimtierfutter-Zusammensetzung nach einem der Ansprüche 1-5 zur Verwendung in einem Verfahren zum Behandeln, Verhindern oder Reduzieren des Risikos der Entwicklung einer metabolischen Endotoxämie bei einem Begleittier, bei dem ein solcher Bedarf besteht.

9. Heimtierfutter-Zusammensetzung zur Verwendung nach einem der Ansprüche 6-8, wobei das Begleittier aus einem Hund und einer Katze ausgewählt ist, bevorzugt ein Hund ist.

## Revendications

1. Composition alimentaire pour animaux de compagnie comprenant :
un complexe de fibres comprenant des fibres détergentes neutres (NDF) en une quantité supérieure à environ 15 % en poids et des fibres brutes en une quantité supérieure à environ 10 % en poids ;
de la protéine en une quantité d'environ 15 % en poids à environ 20 % en poids ; et
de la matière grasse en une quantité d'environ 10 % en poids à environ 14 % en poids ;
dans laquelle le rapport pondéral des NDF à la fibre brute est d'au moins environ 3:2 ;
dans laquelle le complexe de fibres comprend des fibres de noix de pécan, de la pulpe de betterave et/ou du marc de canneberge ; et
dans laquelle tous les poids sont exprimés par rapport au poids total de la composition alimentaire pour animaux de compagnie.

2. Composition alimentaire pour animaux de compagnie selon la revendication 1, dans laquelle les NDF représentent d'environ 55 % à environ 65 % du complexe de fibres.

3. Composition alimentaire pour animaux de compagnie selon la revendication 1 ou la revendication 2, dans laquelle la fibre brute représente environ 35 % à environ 45 % du complexe de fibres.

4. Composition alimentaire pour animaux de compagnie selon l'une quelconque des revendications précédentes, dans laquelle le complexe de fibres représente plus d'environ 25 %, éventuellement plus d'environ 30 %, par rapport au poids total de la composition alimentaire pour animaux de compagnie.

5. Composition alimentaire pour animaux de compagnie selon l'une quelconque des revendications précédentes, comprenant en outre un agent anti-inflammatoire ; éventuellement, dans laquelle l'agent anti-inflammatoire est choisi parmi : la curcu-mine ; la tétrahydrocurcumine ; le clou de girofle ; le gingembre ; le romarin ; le paprika ; une baie (par exemple la myrtille) ; et une combinaison de deux ou plus de ceux-ci.

6. Composition alimentaire pour animaux de compagnie selon l'une quelconque des revendications 1 à 5, pour une utilisation dans une méthode de traitement, de prévention ou d'amélioration d'un symptôme associé à l'obésité chez un animal de compagnie, éventuellement, dans laquelle le symptôme associé à l'obésité est choisi parmi : les douleurs articulaires ; l'inflammation ; et la léthargie.

7. Composition alimentaire pour animaux de compagnie pour une utilisation selon la revendication 6, dans laquelle le symptôme associé à l'obésité est traité, prévenu ou amélioré par augmentation du peptide pancréatique chez l'animal de compagnie.

8. Composition alimentaire pour animaux de compagnie selon l'une quelconque des revendications 1 à 5, pour une utilisation dans une méthode de traitement, de prévention ou de réduction du risque de développer une endotoxémie métabolique chez un animal de compagnie en ayant besoin.

9. Composition alimentaire pour animaux de compagnie pour une utilisation selon l'une quelconque des revendications 6 à 8, dans laquelle l'animal de compagnie est choisi parmi un canidé et un félin, de préférence, un canidé.
